# FASCICULE DE BREVET EUROPEEN

(11) **EP 1 524 968 B1**
(45) Date de publication et mention de la délivrance du brevet: **26.12.2007**
(21) Numéro de dépôt: 03750852.0
(22) Date de dépôt: 28.07.2003
(51) Int. Cl.: A61K 9/50

(54) **FORMULATION PHARMACEUTIQUE ORALE SOUS FORME D'UNE PLURALITE DE MICROCAPSULES PERMETTANT LA LIBERATION PROLONGEE DE PRINCIPE(S) ACTIF(S) PEU SOLUBLE(S)**
ORAL ANZUWENDENDES ARZNEIMITTEL BESTEHEND AUS EINER VIELZAHL VON MIKROKAPSELN FÜR DIE VERZÖGERTE VERABREICHUNG VON WIRKSTOFFEN
ORAL PHARMACEUTICAL FORMULATION IN THE FORM OF A PLURALITY OF MICROCAPSULES FOR PROLONGED RELEASE OF ACTIVE PRINCIPLE(S) WITH LOW SOLUBILITY

(30) Priorité: 26.07.2002 FR 0209532
(43) Date de publication de la demande: 27.04.2005
(73) Titulaire: FLAMEL TECHNOLOGIES, 69200 Vénissieux (FR)
(72) Inventeur: GUIMBERTEAU, Florence, F-33450 Montussan (FR); CASTAN, Catherine, F-69530 Orlienas (FR); MEYRUEIX, Rémi, F-69009 Lyon (FR)
(74) Mandataire: Fleurance, Raphaël
(86) Numéro de dépôt international: PCT/FR2003/002382
(87) Numéro de publication internationale: WO 2004/010983

(56) Documents cités:
- EP-A- 0 709 087
- WO-A-02/39984
- CA-A- 2 068 366
- US-A- 4 894 240
- US-A- 5 084 278
- US-A- 5 286 497

## Description

Le domaine de l'invention est celui de la libération modifiée de principes actifs (PA) pharmaceutiques de faible solubilité.

Dans le présent exposé, l'expression *"libération modifiée",* désigne indifféremment une libération du (ou des) principe(s) actif(s) débutant dès la mise en contact de la forme galénique avec son milieu de dissolution (in vivo ou in vitro) ou bien encore une libération du (ou des) principe(s) actif(s) ne débutant qu'après une durée prédéterminée allant par exemple de 0,5 à plusieurs heures. Ainsi au sens de l'invention, une prolongation de la libération correspond à un temps de libération de 50% du (ou des) principe(s) actif(s) qui est typiquement de plusieurs heures et qui peut s'étendre de 0,25 à 20 heures, par exemple.

L'expression "*faible solubilité"* se rapporte à des principes actifs dont la solubilité dans l'eau est inférieure à 10 g/l à 25°C.

Plus précisément, l'invention concerne des formulations pharmaceutiques à libération prolongée de principes actifs de faible solubilité, cette formulation étant constituée d'une pluralité de microcapsules constituées d'un coeur contenant le principe actif de faible solubilité et enrobé d'une couche de polymère qui contrôle la libération du PA.

Parmi les différents systèmes à libération modifiée, les systèmes galéniques à libération modifiée constitués d'une pluralité de microcapsules de type réservoir de diamètre moyen inférieur à 1000 microns, sont particulièrement avantageux. En effet, dans ces systèmes, la dose de principe(s) actif(s) à administrer se répartit entre grand nombre de microcapsules (typiquement 10000 pour une dose de 500 mg et un diamètre de 400 microns) et ce type de système présente, de ce fait, les avantages intrinsèques suivants :
- La mise en oeuvre d'un mélange de microcapsules de profils de libération modifiée différents, permet de réaliser des profils de libération présentant plusieurs vagues de libération ou assurant par un réglage adéquat des différentes fractions, un niveau de concentration plasmatique du PA constant;
- la sensibilité à la variabilité de la vidange gastrique est moindre, car la vidange, qui s'effectue ici sur un grand nombre de particules est statistiquement plus reproductible;
- On évite la mise en contact des tissus avec une dose élevée en PA « dose dumping ». Chaque microcapsule ne contient en effet qu'une dose très réduite en principe(s) actif(s). On s'affranchit ainsi du risque de détérioration des tissus par surconcentration locale de principe(s) actif(s) agressif;
- Il est possible de combiner plusieurs formes galéniques (libération immédiate et/ou retardée et/ou prolongée) comportant un ou plusieurs principes actifs, dans ces systèmes "multimicrocapsulaires";
- il n'induit pas de dégradation du PA;
- Le temps de séjour des microcapsules dans les parties hautes du tractus peut être prolongé, ce qui assure un accroissement de la durée de passage du (ou des) principe(s) actif(s) devant les fenêtres d'absorption et maximise ainsi la biodisponibilité du (ou des) principe(s) actif(s).

Dans le cas cependant où la solubilité du PA est faible, la réalisation d'une forme microparticulaire à libération modifiée se heurte à une difficulté importante.

La diffusion du principe actif à travers la pellicule d'enrobage entourant chaque microcapsule s'effectue sous l'action du gradient de concentration en PA dissous entre l'intérieur et l'extérieur de la microcapsule. En d'autres termes, c'est la différence de pression osmotique du PA entre l'intérieur et l'extérieur de la microcapsule qui est le moteur de la libération. La concentration interne en PA est la concentration à saturation. La concentration externe en PA est quant à elle négligeable, en conditions usuelles (dites "sink"). Le moteur de la libération est donc directement lié à la concentration à saturation du PA, c'est à dire à sa solubilité.

Pour les PA peu solubles, la concentration à saturation en PA est peu élevée et la diffusion du PA vers l'extérieur est donc a priori très lente, même pour des pellicules d'enrobage peu épaisses.

Et de toutes façons, pour des pellicules d'enrobage de faibles épaisseurs, on se heurte aux difficultés suivantes :
(a) Le dépôt d'une pellicule d'enrobage de très faible épaisseur n'est pas régulier : des lacunes côtoient des zones de surépaisseurs, et la libération du PA n'est pas prolongée.
(b) Le contrôle industriel du procédé de dépôt d'une très faible épaisseur devient très délicat et peu reproductible.

Par ailleurs, pour les pellicules d'enrobage plus épaisses, la libération du PA est extrêmement lente, voire inexistante.

La difficulté à modifier la libération d'un PA peu soluble explique le petit nombre de solutions techniques qui ont été proposées à ce jour.

S'agissant des systèmes galéniques, solides, multimicrocapsulaires, on connaît ceux constitués par une multiplicité de particules ou microcapsules portant chacune du principe(s) actif(s) enrobé d'une couche de pelliculage à base d'éthylcellulose, de polyvinylpyrrolidone, de stéarate de magnésium et d'huile de ricin, par exemple. Un tel système galénique est divulgué dans la demande PCT WO-96/11675. Ces microcapsules-réservoirs tirent de leur multiplicité un avantage, qui est un temps de vidange gastrique plus régulier et reproductible. De plus, leur taille comprise entre 50 et 1 000 µm ainsi que les caractéristiques de leur enrobage permet d'accroître leur temps de transit dans les parties hautes du tractus gastro-intestinal et, par suite, de maintenir l'absorption du principe(s) actif(s) pendant tout ou partie de ce temps de séjour dans l'intestin grêle. Mais, le système galénique multimicrocapsulaire selon le WO-96/11675 est perfectible en ce qui concerne les PA peu solubles administrables oralement, car il ne propose pas de solution au problème de la diffusion d'un tel PA peu soluble à travers une pellicule d'enrobage d'une épaisseur suffisamment importante, par exemple de plusieurs microns.

S'agissant de l'art antérieur sur les microcapsules à libération modifiée de principe actifs peu solubles, il convient de mentionner tout d'abord la demande de brevet PCT WO-99/49846 qui décrit une préparation pharmaceutique composée de particules submicroniques (0,05 à 10 µm) associant un principe actif peu soluble avec un composé phospholipidique, un composé modifiant les charges de surface et un polymère bloc. Cette préparation a pour objectif d'améliorer la biodisponibilité et la stabilité du principe actif et trouve ses applications dans des formes injectables ou bien destinées à être administrées par voie oculaire ou nasale. Une forme à libération prolongée n'est obtenue qu'en cas d'injection intramusculaire.

La demande de brevet PCT WO-00/18374 décrit une invention de même nature que la précédente : le principe actif sous forme de particules submicroniques (< 1000 nm) est stabilisé par un composé associé à la surface des particules et mélangé à un polymère. Ce mélange peut ensuite être mis sous forme de granules ou pellets et éventuellement de comprimé. Le principe actif est rapidement dissous et c'est l'augmentation de la biodisponibilité obtenue grâce à la réduction de taille qui permet d'avoir une concentration plasmatique efficace sur une période prolongée.

La demande de brevet GB-2 202 143 décrit des sphéroïdes de diamètre supérieur à 0,5 mm et de préférence supérieur à 0,8 mm, contenant le principe actif faiblement soluble dispersé dans 70 à 99,5% de cellulose microcristalline. Cette forme matricielle ne requiert aucun enrobage contrôlant la libération du principe actif.

La demande de brevet JP-8073345 décrit un système à libération contrôlée composé d'un granulé pelliculé. Le granulé contient un principe actif faiblement soluble à pH neutre et des acides inorganiques. Ce système propose donc une solution adaptée uniquement au cas des principes actifs basiques faiblement solubles.

Enfin, le brevet européen EP-B-0 249 587 concerne une préparation solide permettant la libération lente d'une substance active, faiblement soluble (< 0,1 % en poids). Cette préparation à libération contrôlée peut se présenter sous forme de gélules comprenant des capsules constituées de granules enrobés. Les granules comprennent le principe actif peu soluble et un solubilisant constitué par le produit commercial Cremophor® RH 40 (huile de ricin hydrogénée polyoxyéthylénée : 40 motifs oxyde d'éthylène), ainsi que d'autres additifs tels que la polyvinylpyrrolidone, la cellulose, l'amidon et le lactose. Ces granules de taille comprise entre 700 et 1120 µm sont revêtus d'une couche d'enrobage en éthylcellulose, permettant le contrôle de la libération. Les ingrédients des granules que sont la polyvinylpyrrolidone, la cellulose, l'amidon de mais et le lactose, semblent être les éléments du système de gel hydrophile propre à la forme galénique selon l'EP-B-0 249 587. Ces capsules comportent donc un seul constituant (éthylcellulose) dans leur couche d'enrobage, ce qui limite ses capacités en matière de modification de la libération du principe actif. En particulier, il est douteux qu'une couche d'enrobage uniquement composée d'éthylcellulose (connue pour former des films imperméables), permette la libération d'un PA peu soluble, de manière contrôlée et industriellement reproductible, sur une durée de quelques heures, par exemple.

Aucune de ces demandes de brevet ne décrit des microparticules de type réservoir ou microcapsules pour lesquelles la libération prolongée du principe actif peu soluble est contrôlée par sa diffusion au travers d'une membrane, ayant une épaisseur suffisante pour assurer une perméabilité contrôlée et industriellement reproductible. Elles n'enseignent pas non plus de quelle façon aboutir à un tel système.

Devant cette vacuité de l'art antérieur, l'un des objectifs essentiels de la présente invention est de proposer une forme à libération modifiée de PA peu soluble(s) constituée d'une pluralité de microcapsules, formée chacune par un coeur contenant le PA et enrobé d'une pellicule d'enrobage.

Un autre objectif de la présente invention est de fournir une pluralité de microcapsules de type réservoir de PA de faible solubilité, pour l'administration orale de ce (ou ces) dernier(s), la pellicule d'enrobage de ces microcapsules ayant une épaisseur suffisante pour assurer une perméabilité contrôlée et industriellement reproductible.

Un autre objectif essentiel de la présente invention est de fournir une pluralité de microcapsules de PA peu soluble(s), de taille inférieure à 1000 µm.

Un autre objectif de la présente invention est de proposer une forme galénique orale et constituée d'un grand nombre (par exemple de l'ordre de plusieurs milliers) de microcapsules, cette multiplicité assurant statistiquement une bonne reproductibilité de la cinétique de transit du PA dans tout le tractus gastro-intestinal, de sorte qu'il en résulte un meilleur contrôle de la biodisponibilité et donc une meilleure efficacité.

Un autre objectif essentiel de la présente invention est de fournir une pluralité de microcapsules de PA peu soluble(s), pour l'administration orale de ce (ou ces) dernier(s) selon un profil de libération prolongée et/ou éventuellement retardée, de telle sorte que le temps de demi-libération t_{1/2} soit compris entre 0,25 et 20 heures.

Un autre objectif essentiel de la présente invention est de fournir une forme orale à libération modifiée dans laquelle le (ou les) PA est (sont) sous forme de pluralité de particules enrobées individuellement pour former des microcapsules et dans laquelle il est possible de mélanger plusieurs principes actifs sous forme multimicrocapsulaire, libérés selon des temps de libération respectifs différents.

S'étant fixé tous les objectifs ci-dessus parmi d'autres, les inventeurs ont eu le mérite de mettre au point un système galénique multimicrocapsulaire à libération prolongée de PA peu solubles:
- qui permette d'ajuster le temps de demi-libération du PA entre 0,25 et 20 heures,
- qui soit reproductible et aisé à mettre en oeuvre industriellement grâce à un rapport de la masse de la pellicule d'enrobage à la masse de la particule, supérieur à 3 % p/p sec, de préférence supérieur à 5 % p/p sec, et, plus préférentiellement encore compris entre 3 et 40 % p/p sec.

Pour ce faire, les inventeurs ont eu le mérite de découvrir après de nombreux essais des microcapsules de structure particulière qui permettent de satisfaire aux objectifs rappelés ci dessus, parmi d'autres.

Ainsi, l'invention concerne des microcapsules permettant la libération modifiée d'au moins un PA peu soluble, destinées à être administrées par voie orale et du type de celles :
- constituées chacune par un coeur comportant au moins un principe actif et par une pellicule d'enrobage appliquée sur le coeur et régissant la libération prolongée du (ou des) PA,
- dont le diamètre moyen est inférieur à 1000 microns, de préférence compris entre 800 et 50 microns et plus préférentiellement encore compris entre 600 et 100 microns,
- dont la pellicule d'enrobage de chaque microcapsule contient les composants suivants :
   → -I-- au moins un polymère filmogène (P1) insoluble dans les liquides du tractus gastro-intestinal
   → -II-- au moins un polymère hydrosoluble (P2)
   → -III- au moins unp1astifiant (PL)
   → -IV- et éventuellement au moins un agent tensioactif (TA) lubrifiant ;
caractérisées en ce que :
➢ leur pellicule d'enrobage représente au moins 3 % p/p sec, de préférence au moins 5 % p/p sec de leur masse totale,
➢ leur coeur contient au moins un PA et au moins un agent solubilisant ayant pour particularité, dès lors qu'il est mis en solution aqueuse à une concentration de 20 % p/p à 37°C, d'accroître de plus de 50 % la solubilité du PA.
➢ le (ou les) agent(s) solubilisant(s) présent(s) dans le coeur avec le PA, confère(nt) au coeur dans lequel il(s) est (sont) inclus des propriétés telles que le comportement du coeur nu (non enrobé) dans un test de dissolution TD donné défini dans les exemples infra, est le suivant : libération de 80 % du PA en moins de deux heures, de préférence en moins d'une heure.

Il est intéressant de noter que cette structure originale de microcapsules avec un coeur comprenant un agent solubilisant et un enrobage à base de P1/P2/PL/(TA), est conçue de manière à améliorer significativement la solubilité du PA en solution aqueuse. Pour parvenir à cela, les microcapsules selon l'invention sont en effet réalisées de telle sorte qu'elles facilitent le mouillage de la surface des cristaux du PA par l'eau.

La solubilité du principe actif est par exemple mesurée en introduisant le PA dans une solution aqueuse contenant l'agent solubilisant. La solution est agitée à 37°C pendant 6 heures puis filtrée sur un filtre de 0,2 µm de diamètre de pore. La teneur en PA solubilisé est évaluée par chromatographie HPLC ou toute autre technique analytique appropriée.

De préférence, les composants P1, P2, PL de la pellicule d'enrobage satisfont aux caractéristiques suivantes :
■ fraction massique en poids sec de P1 par rapport à la masse totale de l' enrobage, comprise entre 40 et 90% et de préférence entre 50 et 80%;
■ fraction massique en poids sec P2/P1+P2 comprise entre 15 et 60 % et de préférence entre 15 et 55%;
■ fraction massique en poids sec PL/P1+PL comprise entre 1 et 30 % et de préférence entre 5 et 25 %.

Selon une variante avantageuse, la pellicule d'enrobage comprend du composant TA à raison de 2 à 20 % et de préférence entre 4 et 15 % de la masse totale de l'enrobage sec.

De préférence, la pellicule d'enrobage représente 3 à 40 % p/p sec de la masse totale des microcapsules.

Conformément à des modalités préférées de réalisation de l'invention, le coeur contenant le PA et l'agent solubilisant peut avoir l'une ou l'autre des structures suivantes :

### Structure A

Le coeur est constitué d'une microbille agréée pour l'administration orale chronique, composée par exemple et de façon non limitative, de dérivés de la cellulose et/ou de composés hydrophiles tels que le sucrose et/ou le dextrose, etc. Ce coeur neutre est recouvert d'une couche comprenant un mélange du PA et d'au moins un agents solubilisants tels que définis ci après.

### Structure B

Le coeur est composé d'un monocristal de PA enrobé par une couche contenant l'agent solubilisant définis ci après.

### Structure C

Le coeur est un granulé composé du mélange du ou des PA, d'un ou plusieurs excipients de granulation connus de l'homme de l'art et d'un ou plusieurs agents solubilisants définis ci après.

Outre le fait qu'ils permettent de résoudre le problème technique à la base de l'invention, l'une des nombreuses caractéristiques avantageuses de ces agents solubilisants est qu'ils appartiennent à la famille des excipients pharmaceutiques agréés pour l'administration orale chronique, par la plupart des autorités compétentes en la matière dans le monde.

De plus, ces solubilisants sont sélectionnés de telle sorte qu'ils n'induisent pas de dégradation du PA.

Ces agents solubilisants sont de préférence choisis de façon non exhaustive parmi les familles suivantes:
(a) les polymères hydrophiles, de préférence :
   - PolyVinylPyrrolidone,
   - Alcool PolyVinylique,
   - dérivés hydrophiles de la cellulose de préférence l'hydroxypropylcellulose et/ou la CarboxyMéthylCellulose,
   - maltodextrines,
   - polyéthylèneglycol (PEG);
(b) les tensioactifs, de préférence :
   - Copolymères polyoxyéthylène-polyoxypropylène,
   - Huile de ricin hydrogénée polyoxyéthylènée,
   - SodiumDodécylSulfate,
   - esters de saccharose et de sorbitan,
   - Phospholipides,
   - Polyéthylène glycol(PEG)-stéarate,
   - Disodiumpamoate,
   - huiles polyoxyéthylénées,
   - polysorbates;
(c) ou encore parmi les séquestrants, de préférence les cyclodextrines ;
(d) et leurs mélanges.

Suivant une caractéristique préférée de l'invention, la fraction massique [agent solubilisant] x 100/ [agent solubilisant + PA] est supérieure ou égale à 5 % et de préférence comprise entre 10 et 98%.

De préférence, P1 est sélectionné dans le groupe de produits suivants :
- les dérivés non hydrosolubles de la cellulose, de préférence l'éthylcellulose et/ou l'acétate de cellulose,
- les dérivés acryliques,
- les polyvinylacétates,
- et leurs mélanges.

De préférence, P2 est sélectionné dans le groupe de produits suivants :
- les dérivés hydrosolubles de la cellulose,
- les polyacrylamides,
- les poly-N-vinylamides,
- les poly-N-vinyl-lactames,
- les alcools polyvinyliques (APV),
- les polyoxyéthylènes (POE),
- les polyvinylpyrrolidones (PVP) (ces dernières étant préférées),
- et leurs mélanges.

De préférence, PL est sélectionné dans le groupe de produits suivants :
- le glycérol et ses esters, de préférence dans le sous-groupe suivant: glycérides acétylés, glycérolmono-stéarate, glycéryltriacétate, glycéroltributyrate,
- les phtalates, de préférence dans le sous-groupe suivant: dibutylphthalate, diéthylphthalate, diméthylphthalate, dioctylphthalate,
- les citrates, de préférence dans le sous-groupe suivant : acétyltributylcitrate, acétyltriéthylcitrate, tributylcitrate, triéthylcitrate,
- les sébaçates, de préférence dans le sous-groupe suivant: diéthylsébaçate, dibutylsébaçate,
- les adipates,
- les azélates,
- les benzoates,
- les huiles végétales,
- les fumarates de préférence le diéthylfumarate,
- les malates, de préférence le diéthylmalate,
- les oxalates, de préférence le diéthyloxalate,
- les succinates; de préférence le dibutylsuccinate,
- les butyrates,
- les esters de l'alcool cétylique,
- l'acide salicylique,
- la triacétine,
- les malonates, de préférence le diéthylmalonate,
- la cutine,
- l'huile de ricin (celle-ci étant particulièrement préférée),
- et leurs mélanges.

De préférence, TA est sélectionné dans le groupe de produits suivants :
- les tensioactifs anioniques, de préférence dans le sous-groupe des sels alcalins ou alcalinoterreux des acides gras, l'acide stéarique et/ou oléique étant préférés,
- et/ou les tensioactifs non ioniques, de préférence dans le sous-groupe suivant:
   o les huiles polyoxyéthylénées de préférence l'huile de ricin hydrogénée polyoxyéthylénée,
   o les copolymères polyoxyéthylène-polyoxypropylène,
   o les esters de sorbitan polyoxyéthylénés,
   o les dérivés de l'huile de ricin polyoxyéthylénés,
   o les stéarates, de préférence de calcium, de magnésium, d'aluminium ou de zinc,
   o les stéarylfumarates, de préférence de sodium,
   o le béhénate de glycérol,
   o et leurs mélanges.

Cette préparation selon l'invention permet de réaliser une forme multimicrocapsulaire à libération modifiée de PA peu solubles, le temps de demi-libération du PA pouvant être ajusté entre 0,25 et 20 heures de façon reproductible grâce à l'utilisation d'une pellicule d'enrobage que l'on peut qualifier de pellicule d'enrobage de diffusion, suffisamment épaisse.

Par ailleurs, pour des PA peu solubles dont la fenêtre d'absorption est limitée, une telle pluralité de microcapsules (typiquement 10 000 pour une dose de 500 mg et un diamètre moyen de 400 microns) présente les avantages intrinsèques suivants :
- La mise en oeuvre d'un mélange de microcapsules de profils de libération retardée et contrôlée différents, permet de réaliser des profils de libération présentant plusieurs vagues de libération ou assurant par un réglage adéquat des différentes fractions, un niveau de concentration plasmatique du PA constant
- La variabilité de la vidange gastrique est moindre, car la vidange qui s'effectue ici sur un grand nombre de particules est statistiquement plus reproductible.
- On évite la mise en contact des tissus avec une dose élevée en PA *"dose dumping".* Chaque microcapsule ne contient en effet qu'une dose très réduite en PA. On s'affranchit ainsi du risque de détérioration des tissus par surconcentration locale de PA agressif.
- Le temps de séjour des microcapsules dans les parties hautes du tractus peut être prolongé, ce qui assure un accroissement de la durée de passage du PA devant les fenêtres d'absorption et maximise ainsi la biodisponibilité du PA.

Les PA peu solubles utilisés pour la préparation des microcapsules à libération modifiée, de préférence contrôlée, selon l'invention peuvent être choisis parmi au moins l'une des grandes variétés de substances actives suivantes :
antiulcéreux, antidiabétiques, anticoagulants, antithrombiques, hypo-lipémiants, antiarythmiques, vasodilatateurs, antiangineux, antihypertenseurs, vasoprotecteurs, promoteurs de fécondité, inducteurs et inhibiteurs du travail utérin, contraceptifs, antibiotiques, antifongiques, antiviraux, anticancéreux, anti-inflammatoires, analgésiques, antiépileptiques, antiparkinsoniens, neuro-leptiques, hypnotiques, anxiolytiques, psychostimulants, anti-migraineux, antidépresseurs, antitussifs, antihistaminiques ou antiallergiques.

De préférence, le ou les PA est (sont) choisi(s) parmi les composés suivants : prazosine, acyclovir, nifedipine, naproxen, ibuprofen, ketoprofen, fenoprofen, indométhacine, diclofenac, sulpiride, terfenadine, carbamazepine, fluoxétine, alprazolam, famotidine, ganciclovir, spironolactone, acide acétylsalycilique, quinidine, morphine, amoxicilline, paracétamol, métoclopramide, vérapamil et leurs mélanges.

S'agissant de la préparation des microcapsules selon l'invention, cela renvoie à des techniques de microencapsulation accessibles à l'homme du métier, dont les principales sont résumées dans l'article de C. DUVERNEY et J. P. BENOIT dans "L'actualité chimique", décembre 1986. Plus précisément, la technique considérée est la microencapsulation par pelliculage, conduisant à des systèmes "réservoir" individualisés par opposition aux systèmes matriciels.
Pour plus de détails, on se réfèrera au brevet EP-B-0 953 359.

Les particules de PA de granulométrie désirée et nécessaire à la réalisation des microcapsules selon l'invention peuvent être des cristaux de PA pur et/ou ayant subi un prétraitement par l'une des techniques conventionnelles en la matière, comme par exemple la granulation, en présence d'au moins un agent liant classique et/ou d'un agent modifiant les caractéristiques de solubilité intrinsèque du PA.

La présente invention vise également un médicament comprenant les microcapsules telles que définies ci-dessus.

Ce médicament peut être sous forme solide : comprimé, gélule, poudre, etc ou sous forme liquide, par exemple suspension aqueuse.

Conformément à l'invention, il est également proposé à titre de solution aux problèmes évoqués au début du présent exposé, à savoir : libération modifiée, de préférence prolongée, de PA peu solubles, dans une forme galénique avalable aisément, le tout dans une perspective de couverture thérapeutique longue, efficace et sûre, d'utiliser une pluralité de microcapsules :
- constituées chacune par un coeur comportant au moins un principe actif et par une pellicule d'enrobage appliquée sur le coeur et régissant la libération prolongée du (ou des) PA,
- dont le diamètre moyen est inférieur à 1000 microns, de préférence compris entre 800 et 50 microns et plus préférentiellement encore compris entre 600 et 100 microns,
- dont la pellicule d'enrobage de chaque microcapsule contient les composants suivants :
   → -I-- au moins un polymère filmogène (P1) insoluble dans les liquides du tractus gastro-intestinal ;
   → -II-- au moins un polymère hydrosoluble (P2);
   → -III- au moins un plastifiant (PL);
   → -IV- et éventuellement au moins un agent tensioactif (TA) lubrifiant ;
caractérisées en ce que :
➢ la pellicule d'enrobage des microcapsules représente au moins 3 % p/p sec, de préférence au moins 5 % p/p sec de la masse totale,
➢ et leur coeur contient au moins un PA et au moins un agent solubilisant ayant pour particularité, dès lors qu'il est mis en solution aqueuse à une concentration de 20 % p/p à 37°C. d'accroître de plus de 50% la solubilité du PA
➢ le (ou les) agent(s) solubilisant(s) présent(s) dans le coeur avec le PA, confère(nt) au coeur dans lequel il(s) est (sont) inclus des propriétés telles que le comportement du coeur nu (non enrobé) dans un test de dissolution TD donné défini dans les exemples infra, est le suivant : libération de 80 % du PA en moins de deux heures, de préférence en moins d'une heure.
pour fabriquer un médicament à base d'au moins un PA peu soluble et administrable par voie orale, avalable aisément et qui se libère in vivo de manière contrôlée, prolongée, et éventuellement retardée.

Selon encore un autre de ses objets, la présente invention concerne une méthode de traitement thérapeutique, dans laquelle on a recours à un médicament tel que défini ci-dessus en tant que produit *per se* ou en tant que produit obtenu par le procédé sus-décrit

L'invention sera mieux comprise sur le plan de sa composition de ses propriétés et de son obtention, à la lecture des exemples ci-après, donnés uniquement à titre d'illustration et permettant de faire ressortir les variantes de réalisation et les avantages de l'invention.

### DESCRIPTION DES FIGURES :

- **La figure 1** représente la courbe du pourcentage de PA dissous (% D) en fonction du temps (t) en heures (H), des microcapsules de l'exemple 1, dans le test de dissolution TD.
- **La figure 2** représente la courbe du pourcentage de PA dissous (% D) en fonction du temps (t) en heures (H), des microcapsules de l'exemple 2, dans le test de dissolution TD.
- **La figure 3** représente la courbe du pourcentage de PA dissous (% D) en fonction du temps (t) en heures (H), des microcapsules de l'exemple 3, dans le test de dissolution TD.

### Exemple 1

### 1.1 - Préparation de microcapsules de Spironolactone :

### Etape 1 : Granulé

180 g de spironolactone, 100 g d'huile de ricin hydrogénée polyoxyéthylénée (40 motifs oxyde d'éthylène), commercialisée sous la marque Cremophor® RH 40 et 120 g de Povidone (Plasdone® K29/32) sont préalablement solubilisés dans un mélange eau / acétone / isopropanol (5/57/38 m/m). Cette solution est ensuite pulvérisée sur 800 g de sphères de cellulose Celphere® CP - 305 (ASAHI KASEI ; diamètre compris entre 300 et 500 µm) dans un appareil à lit d'air fluidisé Glatt® GPC-G1.

### Etape 2 : Enrobage

50 g de granulés obtenus précédemment sont enrobés par 1,60 g d'éthylcellulose (Ethocel® 7 Premium), 0,16 g de Dibutyl-Sébacate, 0,64 g d'huile de ricin hydrogénée polyoxyéthylénée (40 motifs oxyde d'éthylène), commercialisée sous la marque Cremophor® RH 40 et 0,80 g de povidone (Plasdone® K29/32) dissous dans un mélange acétone / isopropanol (60/40 m/m), dans un appareil à lit d'air fluidisé miniGlatt.

### 1-2 - Composition des microcapsules :

**Tableau 1**

| **Ingrédients** | **% massique** | **Formule de fabrication (en g)** |
|---|---|---|
| **Granulés Spironolactone** | **94.00** | |
| - Sphères de cellulose | (62,7) | |
| - Plasdone® K29/32 | (9,4) | 50 |
| - Cremophor® RH 40 | (7,8) | |
| - Spironolactone | (14,1) | |
| **Enrobage** | **6.00** | |
| - Ethocel® 7 Premium | (3,0) | |
| - Plasdone® K29/32 | (1,5) | 3,2 |
| - Cremophor® RH 40 | (1,2) | |
| - Dibutyl Sébacate | (0,3) | |

### 1.3 -Accroissement de la solubilité

Dans une solution aqueuse à pH 6,8 et comportant 20 % en poids de Cremophor® RH 40, la solubilité à 25°C de la spironolactone, qui sans agent solubilisant est de l'ordre de 38 mg/l, est multipliée par un facteur 5.

### 1.4 - Test de Dissolution (TD) :

La cinétique de libération de la spironolactone est déterminée par un test de dissolution (Appareil de type II selon la pharmacopée Européenne 3ème édition, milieu tampon phosphate pH 6,8, volume 1000 ml, température 37°C, agitation palettes 100 tours/min, détection UV 240 nm).

Le test TD est tout d'abord réalisé sur des granulés non enrobés, puis sur des microcapsules comprenant ces granulés.

### Résultat du test TD :

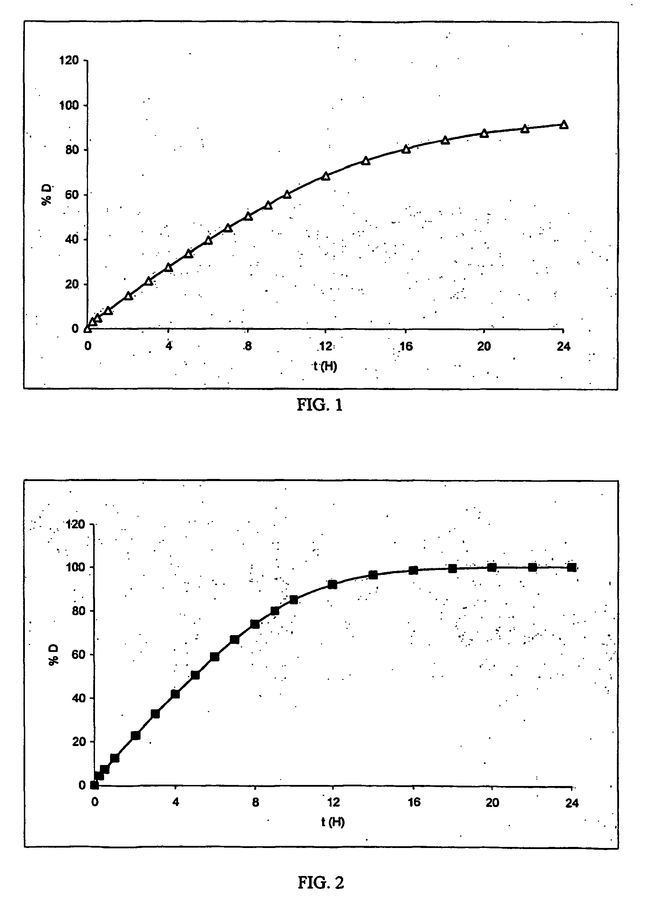

- Granulés non enrobés : la libération est complète (supérieure à 97% dissolution) à t = 1 heure.
- Microcapsules : Le résultat est présenté dans la FIG.1 ci-jointe.

La composition des microcapsules décrite ci-dessus permet d'obtenir un profil à libération modifiée sur 8 h, de spironolactone très faiblement soluble (0,02 g/l). La membrane représente 6% du poids de la microcapsule, ce qui assure un profil de libération reproductible dans un procédé industriel.

### Exemple 2

### 2.1 - Préparation de microcapsules de Spironolactone :

### Etape 1 : Granulé

180 g de spironolactone, 100 g d'huile de ricin hydrogénée polyoxyéthylénée (40 motifs oxyde d'éthylène), commercialisée sous la marque Cremophor® RH 40 et 120 g de Povidone (Plasdone® K29/32) sont préalablement solubilisés dans un mélange eau / acétone / isopropanol (5/57/38 m/m). Cette solution est ensuite pulvérisée sur 800 g de sphères de cellulose (de diamètre compris entre 300 et 500 µm) dans un appareil à lit d'air fluidisé Glatt® GPC-G1.

### Etape 2 : Enrobage

50 g de granulés obtenus précédemment sont enrobés par 1,44 g d'éthylcellulose (Ethocel® 7 Premium), 0,16 g d'huile de ricin, 0,64 g de copolymères polyoxyéthylène-polyoxypropylène (Lutrol® F-68) et 0,96 g de povidone (Plasdone® K29/32) dissous dans un mélange acétone / isopropanol (60/40 m/m), dans un appareil à lit d'air fluidisé miniGlatt®.

### 2.2 - Composition des microcapsules :

**Tableau 2**

| **Ingrédients** | **% massique** | **Formule de fabrication (en g)** |
|---|---|---|
| **Granulés Spironolactone** | **94.00** | |
| - Sphères de cellulose | (62,7) | |
| - Plasdone@K29/32 | (9,4) | 50 |
| - Cremophor® RH 40 | (7,8) | |
| - Spironolactone | (14,1) | |
| **Enrobage** | **6.00** | |
| - Ethocel 7 Premium | (2,7) | |
| - Plasdone K29/32 | (1,8) | 3,2 |
| - Lutrol F-68 | (1,2) | |
| - Huile de ricin | (0,3) | |

### 2.3 - Test TD:

La cinétique de libération de la spironolactone est déterminée par un test de dissolution (Appareil de type II selon la pharmacopée Européenne 3ème édition, milieu tampon phosphate pH 6,8, volume 1000 ml, température 37°C, agitation palettes 100 tours/min, détection UV 240 nm).

### Résultat test TD :

Le résultat est présenté dans la FIG.2 ci-jointe

La composition des microcapsules décrite ci-dessus permet d'obtenir un profil à libération modifiée sur 8 h, de spironolactone très faiblement soluble (0,02 g/l). La membrane représente 6% du poids de la microcapsule, ce qui assure un profil de libération reproductible dans un procédé industriel.

### Exemple 3

### 3.1- Préparation de microcapsules de Spironolactone :

### Etape 1 : Granulé

35 g de spironolactone, 2,5 g d'huile de ricin hydrogénée polyoxyéthylénée (40 motifs oxyde d'éthylène), commercialisée sous la marque Cremophor® RH 40, 12.5 g de Povidone (Plasdone® K29/32) et 200 g de lactose sont préalablement mélangés à sec dans un granulateur de laboratoire (Mi-PRO / Pro-C-ept) pendant 5 minutes. Ce mélange pulvérulent est ensuite granulé à l'eau (20 g). Les granulés sont séchés à 40°C en étuve ventilée, puis calibrés sur grille de 500 µm. On sélectionne par tamisage la fraction 200-500 µm.

### Etape 2 : Enrobage

50 g de granulés obtenus précédemment sont enrobés par 1,88 g d'éthylcellulose (Ethocel® 7 Premium), 0,23 g d'huile de ricin, 0,75 g d'huile de ricin hydrogénée polyoxyéthylénée (40 motifs oxyde d'éthylène), commercialisée sous la marque Cremophor® RH 40 et 0,90 g de povidone (Plasdone® K29/32) dissous dans un mélange acétone / isopropanol (60/40 m/m), dans un appareil à lit d'air fluidisé miniGlatt.

### 3.2 - Composition des microcapsules:

**Tableau 3**

| **Ingrédients** | **% massique** | **Formule de fabrication (en g)** |
|---|---|---|
| **Granulés Spironolactone** | **93.00** | |
| - Lactose | (74,40) | |
| - Plasdone®K29/32 | (4,65) | 50 |
| - Cremophor® RH 40 | (0,93) | |
| - Spironolactone | (13,02) | |
| **Enrobage** | **7.00** | |
| - Ethocel®7 Premium | (3,50) | |
| - Plasdone® K29/32 | (1,68) | |
| - Cremophor® RH 40 | (1,40) | 3,76 |
| - Huile de ricin | (0,42) | |

### 3.3 -Test :

La cinétique de libération de la spironolactone est déterminée par un test de dissolution (Appareil de type II selon la pharmacopée Européenne 3ème édition, milieu tampon phosphate pH 6,8, volume 1000 ml, température 37°C, agitation palettes 100 tours/min, détection UV 240 nm).

### Résultat du test TD :

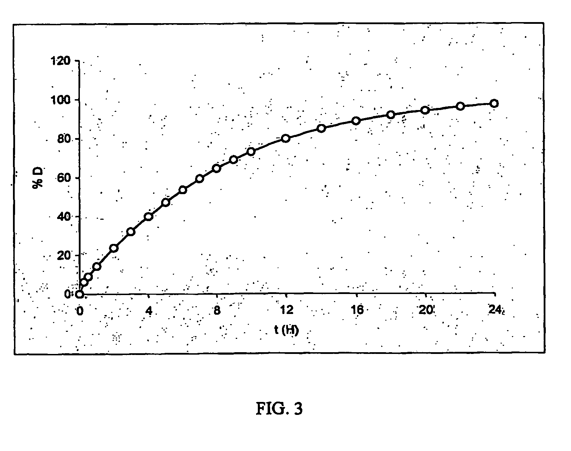

Le résultat est présenté dans la **FIG.3** ci-jointe

La composition des microcapsules décrite ci-dessus permet d'obtenir un profil à libération modifiée sur 12 h, de spironolactone très faiblement soluble (0,02 g/l). La membrane représente 7% du poids de la microcapsule, ce qui assure un profil de libération reproductible dans un procédé industriel.

### Exemple 4

La capacité de l'agent solubilisant formé par le Cremophor® RH 40 à solubiliser un principe actif formé par la spironolactone, est testée selon la méthode de mesure de la solubilité suivante :
Le PA est introduit dans une solutions aqueuse contenant l'agent solubilisant. La solution est agitée à 37°C pendant 6 heures puis filtrée sur un filtre de 0,2 µm de diamètre de pore. La teneur en PA solubilisé est évaluée par HPLC. Les résultats sont donnés dans le tableau 4 ci-après.

**Tableau 4**

| **Crémophor® RH 40 % p/p à 37° C** | **(Spironolactone) dissoute en solution mg/ml** |
|---|---|
| 0 | < 0,1 |
| 1 | 0,1 |
| 2 | 0,2 |
| 5 | 0,4 |
| 10 | 0,8 |
| 20 | 1,4 |

## Revendications

1. Microcapsules permettant la libération modifiée d'au moins un principe actif (PA) peu soluble dont la solubilité dans l'eau est inférieure à 10 g/l à 25°C, destinées à être administrées par voie orale et du type de celles :
• constituées chacune par un coeur comportant au moins un principe actif et par une pellicule d'enrobage appliquée sur le coeur et régissant la libération modifiée du (ou des) PA,
• dont le diamètre moyen est inférieur à 1000 microns, de préférence compris entre 800 et 50 microns et plus préférentiellement encore compris entre 600 et 100 microns,
• dont la pellicule d'enrobage de chaque microcapsule contient les composants suivants:
→ -I- au moins un polymère filmogène (P1) insoluble dans les liquides du tractus gastro-intestinal ;
→ -II- au moins un polymère hydrosoluble (P2) ;
→ -III- au moins un plastifiant (PL) ;
→ -IV- et éventuellement au moins un agent tensioactif (TA) lubrifiant ;
**caractérisées en ce que** :
➢ leur pellicule d'enrobage représente au moins 3 % p/p sec, de préférence au moins 5% p/p sec de leur masse totale,
➢ leur coeur contient au moins un PA et au moins un agent solubilisant ayant pour particularité, dès lors qu'il est mis en solution aqueuse à une concentration de 20 % p/p à 37°C, d'accroître de plus de 50% la solubilité du PA,
➢ le (ou les) ageng(s) solubilisant(s) présent(s) dans le coeur avec le PA, confère(nt) au coeur dans lequel il(s) est (sont) inclus des propriétés telles que le comportement du coeur nu (non enrobé) dans un test de dissolution TD donné, est le suivant : libération de 80 % du PA en moins de deux heures, de préférence en moins d'une heure.

2. Microcapsules selon la revendication 1, **caractérisées en ce que** les composants P1, P2, PL de la pellicule d'enrobage satisfont aux caractéristiques suivantes :
■ fraction massique en poids sec de P1 par rapport à la masse totale de l'enrobage, comprise entre 40 et 90 % et de préférence entre 50 et 80 %;
■ fraction massique en poids sec P2/P1+P2 comprise entre 15 et 60 % et de préférence entre 15 et 55 %;
■ fraction massique en poids sec PL/P1+PL comprise entre 1 et 30 % et de préférence entre 5 et 25%.

3. Microcapsules selon la revendication 1 ou 2, **caractérisées en ce que** la pellicule d'enrobage comprend du composant TA à raison de 2 à 20 % et de préférence entre 4 et 15% de la masse totale de l'enrobage sec.

4. Microcapsules selon l'une quelconque des revendications 1 à 3, **caractérisées en ce que** l'agent solubilisant est choisi parmi les familles suivantes :
(a) les polymères hydrophiles, de préférence :
- PolyVinylPyrrolidone,
- Alcool polyVinylique,
- dérivés hydrophiles de la cellulose de préférence l'hydroxypropylcellulose et/ou la-CarboxyMéthylCellulose,
- Maltodextrines,
- polyéthylèneglycol (PEG);
(b) les tensioactifs, de préférence :
- Copolymères polyoxyéthylène-polyoxypzopylène,
- Huile de ricin hydrogénée polyoxyéthylènés,
- SodiumDodécylSulfate,
- esters de saccharose et de sorbitan,
- Phospholipides,
- Polyéthylène glycol(PEG)-stéarate,
- Disodiumpamoate,
- huiles polyoxyéthylénées,
- polysorbates;
(c) ou encore parmi les séquestrants, de préférence les cyclodextrines ;
(d) et leurs mélanges.

5. Microcapsules selon l'une quelconque des revendications 1 à 4, **caracterisées en ce que** la fraction massique [agent solubilisant] x 100/ [agent solubilisant + PA] est supérieure ou égale à 5 % et de préférence comprise entre 10 et 98%.

6. Microcapsules selon l'une quelconque des revendications 1 à 5, **caractérisées en ce que** P1 est sélectionné dans le groupe de produits suivants:
• les dérivés non hydrosolubles de la cellulose, de préférence l'éthylcellulose et/ou l'acétate de cellulose,
• les dérivés acryliques,
• les polyvinylacétates,
• et leurs mélanges.

7. Microcapsules selon l'une quelconque des revendications 1 à 6, **caractérisées en ce que** P2 est sélectionné dans le groupe de produits suivants:
• les dérivés hydrosolubles de la cellulose,
• les polyacrylamides,
• les poly-N-vinylamides,
• les poly-N-vinyl-lactames,
• les alcools polyvinyliques (APV),
• les Pelyoxyéthylènes (POE),
• les polyvinylpyrrolidones (PVP) (ces dernières étant préférées),
• et leurs mélanges.

8. Microcapsules selon l'une quelconque des revendications 1 à 7, **caractérisées en ce que** PL est sélectionné dans le groupe de produits suivants:
• le glycérol et ses esters, de préférence dans le sous-groupe suivant: glycérides acétylés, glycérolmonostéarate, glycéryltriacétate, glycéroltributyrate,
• les phtalates, de préférence dans le sous-groupe suivant : dibutylphthalate, diéthylphthalate, diméthylphthalate, dioctyl-phthalate,
• les citrates, de préférence dans le sous-groupe suivant : acétyltributylcitrate, acétyltriéthylcitrate, tributylcitrate, triéthyl-citrate,
• les sébaçates, de préférence dans le sous-groupe suivant: diéthylsébaçate, dibutylsébaçate,
• les adipates,
• les azélates,
• les benzoates,
• les huiles végétales,
• les fumarates de préférence le diéthylfumarate,
• les malates; de préférence le diéthylmalate,
• les oxalates, de préférence le diéthyloxalate,
• les succinates; de préférence le dibutylsuccinate,
• les butyrates,
• les esters de l'alcool cétylique,
• l'acide salicylique,
• la triacétine,
• les malonates, de préférence le diéthylmalonate,
• la cutine,
• l'huile de ricin (celle-ci étant particulièrement préférée),
• et leurs mélanges.

9. Microcapsules selon l'une quelconque des revendications 1 à 8, **caractérisées en ce que** TA est sélectionné dans le groupe de produits suivants:
• les tensioactifs anioniques, de préférence dans le sous-groupe des sels alcalins ou alcalinoterreux des acides gras, l'acide stéarique et/ou oléique étant préférés,
• et/ou les tensioactifs non ioniques, de préférence dans le sous-groupe suivant:
o les huiles polyoxyéthyiénées de préférence l'huile de ricin hydrogénée polyoxyéthylénée,
o les copolymères polyoxyéthylène-polyoxypropylène,
o les esters de sorbitan polyoxyéthylénés,
o les dérivés de l'huile de ricin polyoxyéthylénés,
o les stéarates, de préférence de calcium, de magnésium, d'aluminium ou de zinc,
o les stéarylfumarates, de préférence de sodium,
o le béhénate de glycérol,
o et leurs mélanges.

10. Microcapsules selon l'une quelconque des revendications 1 à 9, **caractérisées en ce que** les PA peu solubles sont choisis parmi au moins l'une des grandes variétés de substances actives suivantes :
antiulcéreux, antidiabétiques, anticoagulants, antithrombiques, hypo-lipémiants, antiarythmiques, vasodilatateurs, antiangineux, antihypertenseurs, vasoprotecteurs, promoteurs de fécondité, inducteurs et inhibiteurs du travail utérin, contraceptifs, antibiotiques, antifongiques, antiviraux, anticancéreux, anti-inflammatoires, analgésiques, antiépileptiques, antiparkinsoniens, neuroleptiques, hypnotiques, anxiolytiques, psychostimulants, antimigraineux, antidépresseurs, antitussifs, antihistaminiques ou antiallergiques.

11. Microcapsules selon la revendication 10, **caractérisées en ce que** le (ou les) PA peu soluble(s) est (sont) choisi(s) parmi les composés suivants : prazosine, acyclovir, nifedipine, naproxen, ibuprofen, ketoprofen, fenoprofen, indométhacine, diclofenac, sulpiride, terfenadine, carbamazepine, fluoxétine, alprazolam, famotidine, ganciclovir, spironolactone, acide acétylsalycilique, quinidine, morphine, amoxicilline, paracétamol, métoclo-pramide, vérapamil et leurs mélanges.

12. Médicament comprenant les microcapsules selon l'une quelconque des revendications 1 à 11.

13. Médicament selon la revendication 12, **caractérisé en ce qu'**il est sous forme solide de préférence : comprimé, gélule ou poudre, ou sous forme liquide, de préférence : suspension aqueuse.

14. Utilisation de microcapsules :
• constituées chacune par un coeur comportant au moins un principe actif et par une pellicule d'enrobage appliquée sur le coeur et régissant la libération prolongée du (ou des) PA,
• dont le diamètre moyen est inférieur à 1000 microns, de préférence compris entre 800 et 50 microns et plus préférentiellement encore compris entre 600 et 100 microns,
• dont la pellicule d'enrobage de chaque microcapsule contient les composants suivants :
→ -I-- au moins un polymère filmogène (P1) insoluble dans les liquides du tractus gastro-intestinal;
→ -II- au moins un polymère hydrosoluble (P2) ;
→ -III- au moins un plastifiant (PL);
→ -IV- et éventuellement au moins un agent tensioactif (TA) lubrifiant ;
**caractérisées en ce que** :
➢ leur pellicule d'enrobage représente au moins 4 % p/p sec, de préférence au moins 5 % p/p sec de leur masse totale,
➢ leur coeur contient au moins un PA et au moins un agent solubilisant ayant pour particularité, dès lors qu'il est mis en solution aqueuse à une concentration de 20 % p/p à 37°C, d'accroître de plus de 50% la solubilité du PA,
➢ le (ou les) agent(s) solubilisant(s) présent(s) dans le coeur avec le PA, confère(nt) au coeur dans lequel il(s) est (sont) inclus des propriétés telles que le comportement du coeur nu (non enrobé) dans un test de dissolution TD donné, est le suivant : libération de 80 % du PA en moins de deux heures, de préférence en moins d'une heure,
pour fabriquer un médicament à base d'au moins un PA peu soluble dont la solubilité dans l'eau est inférieure à 10 g/l à 25°C, et administrable par voie orale, avalable aisément et qui se libère in vivo de manière contrôlée, prolongée, et éventuellement retardée.

## Claims

1. Microcapsules for the modified release of at least one AP with low solubility, the water-solubility of which is less than 10 g/l at 25°C, intended to be administered orally and of the type of those:
• each consisting of a core comprising at least one active principle and of a coating film applied onto the core and controlling the modified release of the AP(s),
• the mean diameter of which is less than 1000 microns, preferably between 800 and 50 microns, and even more preferably between 600 and 100 microns,
• in which the coating film of each microcapsule contains the following components:
→ -I-- at least one film-forming polymer (P1) insoluble in gastrointestinal tract fluids,
→ -II-- at least one water-soluble polymer (P2),
→ -III- at least one plasticizer (PL),
→ -IV- and, optionally, at least one lubricating surfactant (TA) ;
**characterized in that**:
➢ their coating film represents at least 3% dry weight/dry weight, preferably at least 5% dry weight/dry weight of their total mass,
➢ their core contains at least one AP and at least one solubilizing agent having the particularity, as soon as it is placed in aqueous solution at a concentration of 20% w/w at 37°C, of increasing the solubility of the AP by more than 50%,
➢ the solubilizing agent(s) present in the core with the AP confer(s), on the core in which it (they) is (are) included, properties such that the behavior of the exposed (non-coated) core in a given dissolving test TD is as follows: release of 80% of the AP in less than two hours, preferably in less than one hour.

2. The microcapsules as claimed in claim 1, **characterized in that** the components P1, P2 and PL of the coating film satisfy the following characteristics:
■ mass fraction by dry weight of P1 relative to the total mass of the coating of between 40 and 90%, and preferably of between 50 and 80%;
■ mass fraction by dry weight P2/P1+P2 of between 15 and 60%, and preferably of between 15 and 55%;
■ mass fraction by dry weight PL/P1+PL of between 1 and 30%, and preferably of between 5 and 25%.

3. The microcapsules as claimed in claim 1 or 2, **characterized in that** the coating film comprises component TA in a proportion of 2 to 20%, and preferably of between 4 and 15% of the total mass of the dry coating.

4. The microcapsules as claimed in any one of claims 1 to 3, **characterized in that** the solubilizing agent is chosen from the following families:
(a) hydrophilic polymers, preferably:
- polyvinyl pyrrolidone,
- polyvinyl alcohol,
- hydrophilic derivatives of cellulose, preferably hydroxypropylcellulose and/or carboxymethylcellulose,
- maltodextrins,
- polyethylene glycol (PEG) ;
(b) surfactants, preferably:
- polyoxyethylene-polyoxypropylene copolymers,
- polyoxyethylenated hydrogenated castor oil,
- sodium dodecyl sulfate,
- esters of sucrose and of sorbitan,
- phospholipids,
- polyethylene glycol (PEG) stearate,
- disodium pamoate,
- polyoxyethylenated oils,
- polysorbates;
(c) or else from sequestering agents, preferably cyclodextrins;
(d) and mixtures thereof.

5. The microcapsules as claimed in any one of claims 1 to 4, **characterized in that** the mass fraction [solubilizing agent] x 100/ [solubilizing agent + AP] is greater than or equal to 5%, and preferably between 10 and 98%.

6. The microcapsules as claimed in any one of claims 1 to 5, **characterized in that** P1 is selected from the group of products below:
• water-insoluble derivatives of cellulose, preferably ethylcellulose and/or cellulose acetate,
• acrylic derivatives,
• poly(vinyl acetates),
• and mixtures thereof.

7. The microcapsules as claimed in any of one of claims 1 to 6, **characterized in that** P2 is selected from the group of products below:
• water-soluble derivatives of cellulose,
• polyacrylamides,
• poly-N-vinylamides,
• poly(N-vinyl lactams),
• polyvinyl alcohols (PVAs),
• polyoxyethylenes (POEs),
• polyvinylpyrrolidones (PVPs) (the latter being preferred),
• and mixtures thereof.

8. The microcapsules as claimed in any one of claims 1 to 7, **characterized in that** PL is selected from the group of products below:
• glycerol and esters thereof, preferably from the following subgroup: acetylated glycerides, glyceryl monostearate, glyceryl triacetate, glyceryl tributyrate,
• phthalates, preferably from the following subgroup:
dibutyl phthalate, diethyl phthalate, dimethyl phthalate, dioctyl phthalate,
• citrates, preferably from the following subgroup:
acetyl tributyl citrate, acetyl triethyl citrate, tributyl citrate, triethyl citrate,
• sebacates, preferably from the following subgroup:
diethyl sebacate, dibutyl sebacate,
• adipates,
• azelates,
• benzoates,
• plant oils,
• fumarates, preferably diethyl fumarate,
• malates, preferably diethyl malate,
• oxalates, preferably diethyl oxalate,
• succinates, preferably dibutyl succinate,
• butyrates,
• cetyl alcohol esters,
• salicylic acid,
• triacetin,
• malonates, preferably diethyl malonate,
• cutin,
• castor oil (this being particularly preferred),
• and mixtures thereof.

9. The microcapsules as claimed in any one of claims 1 to 8, **characterized in that** TA is selected from the group of products below:
• anionic surfactants, preferably from the subgroup of alkali metal salts or alkaline-earth metal salts of fatty acids, stearic acid and/or oleic acid being preferred,
• and/or nonionic surfactants, preferably from the following subgroup:
o polyoxyethylenated oils, preferably polyoxyethylenated hydrogenated castor oil,
o polyoxyethylene-polyoxypropylene copolymers,
o polyoxyethylenated sorbitan esters,
o polyoxyethylenated castor oil derivatives,
o stearates, preferably calcium stearate, magnesium stearate, aluminum stearate or zinc stearate,
o stearyl fumarates, preferably sodium stearyl fumarate,
o glyceryl behenate,
o and mixtures thereof.

10. The microcapsules as claimed in any one of claims 1 to 9, **characterized in that** the APs with low solubility are chosen from at least one of the major varieties of active substances below:
antiulcer agents, antidiabetic agents, anticoagulants, antithrombics, blood lipid-lowering agents, anti-arrhythmics, vasodilators, antiangina agents, antihypertensives, vasoprotective agents, fertility promoters, inducers and inhibitors of uterine labor, contraceptives, antibiotics, antifungal agents, antiviral agents, anticancer agents, anti-inflammatories, analgesics, antiepileptics, antiparkinsonian agents, neuroleptics, hypnotics, anxiolytics, psychostimulants, antimigraine agents, antidepressives, antitussives, antihistamines or antiallergic agents.

11. The microcapsules as claimed in claim 10, **characterized in that** the AP(s) with low solubility is (are) chosen from the following compounds: prazosine, acyclovir, nifedipine, naproxen, ibuprofen, ketoprofen, fenoprofen, indomethacine, diclofenac, sulpiride, terfenadine, carbamazepine, fluoxetine, alprazolam, famotidine, ganciclovir, spironolactone, acetylsalicyclic acid, quinidine, morphine, amoxicillin, paracetamol, metoclopramide, verapamil and mixtures thereof.

12. A medicinal product comprising the microcapsules as claimed in any one of claims 1 to 11.

13. The medicinal product as claimed in claim 12, **characterized in that** it is in solid form, preferably: tablet, gelatin capsule or powder, or in liquid form, preferably: an aqueous suspension.

14. The use of microcapsules:
• each consisting of a core comprising at least one active principle and of a coating film applied onto the core and controlling the prolonged release of the AP(s),
• the mean diameter of which is less than 1000 microns, preferably between 800 and 50 microns, and even more preferably between 600 and 100 microns,
• in which the coating film of each microcapsule contains the following components:
→ -I-- at least one film-forming polymer (P1) insoluble in gastrointestinal tract fluids,
→ -II-- at least one water-soluble polymer (P2),
→ -III- at least one plasticizer (PL),
→ -IV- and, optionally, at least one lubricating surfactant (TA);
**characterized in that**:
➢ their coating film represents at least 4% dry weight/dry weight, preferably at least 5% dry weight/dry weight of their total mass,
➢ their core contains at least one AP and at least one solubilizing agent having the particularity, as soon as it is placed in aqueous solution at a concentration of 20% w/w at 37°C, of increasing the solubility of the AP by more than 50%,
➢ the solubilizing agent(s) present in the core with the AP confer(s), on the core in which it (they) is (are) included, properties such that the behavior of the exposed (non-coated) core in a given dissolving test TD is as follows: release of 80% of the AP in less than two hours, preferably in less than one hour,
for producing a medicinal product based on at least one AP with low solubility, the water-solubility of which is less than 10 g/l at 25, which can be administered orally, which can be readily swallowed, and which is released in vivo in a controlled, prolonged and, optionally, delayed manner.

## Patentansprüche

1. Mikrokapseln, die eine modifizierte Freisetzung mindestens eines schlecht löslichen Wirkstoffs (PA), dessen Löslichkeit in Wasser unter 10 g/l bei 25°C liegt erlauben, darauf ausgerichtet, auf oralem Wege verabreicht zu werden, und folgender Art:
- jeweils aus einem Kern bestehend, der mindestens einen Wirkstoff umfasst und mit einer Hüllschicht, die auf den Kern aufgetragen wird, und die modifizierte Freisetzung des (oder der) PA steuert,
- deren mittlerer Durchmesser unter 1.000 µm liegt, vorzugsweise zwischen 800 und 50 µm liegt, und noch bevorzugter zwischen 600 und 100 µm liegt,
- wobei die Hüllschicht jeder Mikrokapsel die folgenden Bestandteile enthält:
I. mindestens ein filmbildendes Polymer (P1), das in den Flüssigkeiten des Gastrointestinaltrakts unlöslich ist;
II. mindestens ein wasserlösliches Polymer (P2) ;
III. mindestens einen Weichmacher (PL) ;
IV. und gegebenenfalls mindestens ein oberflächenaktives Schmiermittel (TA) ;
**dadurch gekennzeichnet, dass**:
- ihre Hüllschicht mindestens 3 Gew.-% Trockengewicht, vorzugsweise mindestens 5 Gew.-% Trockengewicht ihrer gesamten Masse darstellt,
- ihr Kern mindestens einen PA und mindestens ein Lösungsmittel enthält, das die Eigenschaft besitzt, dass es, sofern es in eine wässrige Lösung in einer Konzentration von 20 Gew.-% bei 37°C gegeben wird, die Löslichkeit des PA um mehr als 50 % erhöht,
- das (oder die) Lösungsmittel, die in dem Kern zusammen mit dem PA vorhanden sind, dem Kern, in dem es/sie enthalten ist/sind, Eigenschaften, wie das Verhalten des nackten (nicht umhüllten) Kerns in einem gegebenen Auflösungstest TD verleihen, der wie folgt ist: Freisetzung von 80 % des PA in weniger als zwei Stunden, vorzugsweise weniger als einer Stunde.

2. Mikrokapseln gemäß Anspruch 1, **dadurch gekennzeichnet, dass** die Bestandteile P1, P2, PL der Hüllschicht die folgenden Eigenschaften erfüllen:
Anteil des Trockenmassegewichts von P1, bezogen auf die gesamte Masse der Hülle, zwischen 40 und 90 % und bevorzugt zwischen 50 und 80 %;
Anteil der Trockenmassegewichts P2/P1+P2 zwischen 15 und 60 % und vorzugsweise zwischen 15 und 55 %;
Anteil des Trockenmassegewichts PL/P1+PL zwischen 1 und 30 % und vorzugsweise zwischen 5 und 25 %.

3. Mikrokapseln gemäß Anspruch 1 oder 2, **dadurch gekennzeichnet, dass** die Hüllschicht den Bestandteil TA in einem Verhältnis von 2 bis 20 % und vorzugsweise zwischen 4 und 15 % der Gesamtmasse des Trockengewichts der Hüllschicht umfasst.

4. Mikrokapseln gemäß irgendeinem der Ansprüche 1 bis 3, **dadurch gekennzeichnet, dass** das Lösungsmittel aus den folgenden Familien ausgewählt ist:
(a) hydrophilen Polymeren, vorzugsweise:
Polyvinylpyrrolidon,
Polyvinylalkohol,
hydrophile Cellulosederivate, vorzugsweise der
Hydroxypropylcellulose und/oder der
Carboxymethylcellulose,
Maltodextrine,
Polyethylenglycol (PEG);
(b) Tensiden, vorzugsweise:
Polyoxyethylen-Polypropylen-Copolymeren, hydrogeniertes polyoxyethyleniertes Rizinöl,
Natriumdodecylsulfat,
Sorbitansaccharoseester,
Phospholipide,
Polyethylenglycol-(PEG)-stearat,
Dinatriumpamoat,
polyoxyethylenierte Öle,
Polysorbate;
(c) oder aus Komplexbildnern, vorzugsweise Cyclodextrin;
(d) und ihren Gemischen.

5. Mikrokapseln gemäß irgendeinem der Ansprüche 1 bis 4, **dadurch gekennzeichnet, dass** der Masseanteil (Lösungsmittel) x 100/(Lösungsmittel + PA) über oder gleich 5 % ist und vorzugsweise zwischen 10 und 98 % liegt.

6. Mikrokapseln gemäß irgendeinem der Ansprüche 1 bis 5, **dadurch gekennzeichnet, dass** P1 aus der Gruppe der folgenden Produkte ausgewählt ist:
wasserunlösliche Cellulosederivate, vorzugsweise Ethylcellulose und/oder Celluloseacetat, Acrylderivate,
Polyvinylacetate,
und deren Gemische.

7. Mikrokapseln gemäß irgendeinem der Ansprüche 1 bis 6, **dadurch gekennzeichnet, dass** P2 aus der Gruppe der folgenden Produkte ausgewählt ist:
wasserlöslichen Cellulosederivaten,
Polyacrylamiden,
Poly-N-vinylamiden,
Poly-N-vinyllactamen,
Polyvinylalkoholen (PVA),
Polyoxyethylenen (POE),
Polyvinylpyrrolidonen (PVP) (die letztgenannten sind bevorzugt), und deren Gemischen.

8. Mikrokapseln gemäß irgendeinem der Ansprüche 1 bis 7, **dadurch gekennzeichnet, dass** PL ausgewählt ist aus der Gruppe der folgenden Produkte:
Glycerol und dessen Estern, vorzugsweise aus der folgenden Untergruppe: acetylierte Glyceride, Glycerolmonostearat, Glyceryltriacetat, Glyceroltributyrat,
den Phthalaten, vorzugsweise aus der folgenden Untergruppe: Dibutylphthalat, Diethylphthalat, Dimethylphthalat, Dioctylphthalat,
den Citraten, vorzugsweise aus der folgenden Untergruppe: Acetyltributylcitrat, Acetyltriethylcitrat, Tributylcitrat, Triethylcitrat,
den Sebacaten, vorzugsweise aus der folgenden Gruppe: Diethylsebacat, Dibutylsebacat,
den Adipaten,
den Azelaten,
den Benzoaten,
den Pflanzenölen,
den Fumaraten, vorzugsweise Diethylfumarat,
den Malaten, vorzugsweise Diethylmalat,
den Oxalaten, vorzugsweise Diethyloxalat,
den Succinaten, vorzugsweise Dibutylsuccinat,
den Butyraten,
den Cetylalkoholestern,
der Salicylsäure,
dem Triacetin,
den Malonaten, vorzugsweise Diethylmalonat,
dem Cutin,
Rizinöl (dieses ist besonders bevorzugt), und ihren Gemischen.

9. Mikrokapseln gemäß irgendeinem der Ansprüche 1 bis 8, **dadurch gekennzeichnet, dass** TA aus der folgenden Produktgruppe ausgewählt ist:
den anionischen Tensiden, vorzugsweise aus der Untergruppe Alkalisalzen oder Erdalkalisalzen der Fettsäuren, wobei Stearinsäure und/oder Oleinsäure bevorzugt sind,
und/oder den nichtionischen Tensiden, vorzugsweise aus der folgenden Untergruppe: polyoxyethylenierten Ölen, vorzugsweise polyoxyethyliertem hydrogeniertem Rizinöl, den Polyoxyethylen-Polyoxypropylen-Copolymeren,
den Polyoxyethylensorbitanestern,
den Derivaten des polyoxyethylenierten Rizinöls,
den Stearaten, vorzugsweise von Calcium, Magnesium, Aluminium oder Zink,
den Stearylfumaraten, vorzugsweise des Natriums,
dem Glycerolbenenat,
und deren Gemischen.

10. Mikrokapseln gemäß irgendeinem der Ansprüche 1 bis 9, **dadurch gekennzeichnet, dass** die schlecht löslichen PA ausgewählt sind aus mindestens einer der großen Anzahl an folgenden Wirkstoffen: Antiulcerativa, Antidiabetika, Antikoagulanzien, Antithrombotika, Fettsenkern, Antiarythmiemitteln, Vasodilatatoren, Mitteln gegen Angina, Mitteln gegen Hochdruck, gefäßschützenden Mitteln, Fertilitäts-fördernden Mitteln, Förderern oder Inhibitoren von Wehen, Kontrazeptiva, Antibiotika, Antifungiziden, Antivirusmitteln, Antikrebsmitteln, Anti-Entzündungsmitteln, Analgetika, Antiepileptika, Anti-Parkinson-Mitteln, Neuroleptika, Hypnotika, Anxiolytika, Psychostimulanzien, Anti-Migräne-Mittel, Antidepressiva, Antitussiva, Antihistaminika oder Antiallergika.

11. Mikrokapseln gemäß Anspruch 10, **dadurch gekennzeichnet, dass** der (oder die) schwach lösliche(n) PA ausgewählt sind aus den folgenden Verbindungen: Prazosin, Acyclovir, Nifedipin, Naproxen, Ibuprofen, Ketoprofen, Fenoprofen, Indomethacin, Diclofenac, Sulpiridin, Terfenadin, Carbamazepin, Fluoxetin, Alprazolam, Famotidin, Gancyclovir, Spironolacton, Acetylsalicylsäure, Chinidin, Morphin, Amoxicillin, Paracetamol, Metoclopramid, Verapamil und ihren Gemischen.

12. Medikament, umfassend die Mikrokapseln gemäß irgendeinem der Ansprüche 1 bis 11.

13. Medikament gemäß Anspruch 12, **dadurch gekennzeichnet, dass** es in fester Form vorliegt, vorzugsweise: Tablette, Kapsel oder Pulver, oder in flüssiger Form, vorzugsweise in wässriger Suspension.

14. Verwendung von Mikrokapseln:
jeweils aus einem Kern, der mindestens einen Wirkstoff umfasst, und einer Hüllschicht gebildet, die auf den Kern aufgetragen ist, und die verlängerte Freisetzung des (oder der) PAs reguliert,
wobei der mittlere Durchmesser unter 1.000 µm liegt, vorzugsweise zwischen 800 und 50 µm liegt und noch bevorzugter zwischen 600 und 100 µm liegt,
wobei die Hüllschicht jeder Mikrokapsel die folgenden Bestandteile enthält:
I. mindestens ein filmbildendes Polymer (P1), welches in den Flüssigkeiten des Gastrointestinaltrakts unlöslich ist;
II. mindestens ein wasserlösliches Polymer (P2);
III. mindestens einen Weichmacher (PL);
IV. und gegebenenfalls mindestens ein tensioaktives Schmiermittel (TA) ;
**dadurch gekennzeichnet, dass** ihre Hüllschicht mindestens 4 % des Trockengewichts ausmacht, vorzugsweise mindestens 5 % des Trockengewichts ihrer gesamten Masse, ihr Kern mindestens ein PA und mindestens ein Lösungsmittel enthält, das die Eigenschaft besitzt, dass es, wenn es in eine wässrige Lösung in einer Konzentration von 20 Gew.-% bei 37°C gegeben wird, die Löslichkeit des PA um mehr als 50 % erhöht,
das (oder die) Lösungsmittel, das (die) in dem Kern mit dem PA vorhanden sind dem Kern, in dem er/sie enthalten ist/sind die Eigenschaften des nackten Kerns (nicht umhüllt) in einem gegebenen Auflösungstest verleihen, welcher wie folgt ist: Freisetzung von 80 % des Wirkstoffs in weniger als zwei Stunden, vorzugsweise weniger als einer Stunde,
zur Herstellung eines Medikaments auf Grundlage mindestens eines schlecht löslichen PA, dessen Löslichkeit in Wasser unter 10 g/l bei 25°C liegt, und auf oralem Wege verabreichbar ist, leicht schluckbar ist, und sich in vivo in kontrollierter Form, verlängerter Form und gegebenenfalls verzögerter Form freisetzt.
